# EUROPEAN PATENT APPLICATION

(11) **EP 2 551 355 A1**
(43) Date of publication of application: **30.01.2013**
(21) Application number: 11176025.2
(22) Date of filing: 29.07.2011
(51) Int. Cl.: C12Q 1/68

(54) **Method for identifying a cholestatic compound**

(71) Applicant: Universiteit Maastricht, 6211 LK Maastricht (NL); Academisch Ziekenhuis Maastricht, 6229 HX Maastricht (NL); Rijksinstituut Voor Volksgezondheid En Milieu, 3721 MA Bilthoven (NL); Rikilt, 6708 WB Wageningen (NL)
(72) Inventor: Kienhuis, Anne Susan, 3720 BA BILTHOVEN (NL); Pennings, Jeroen Lambertus Antonius, 3720 BA BILTHOVEN (NL); Pronk, Theresia Elizabeth, 3720 BA BILTHOVEN (NL); van der Ven, Leonardus Theodorus Maria, 3720 BA BILTHOVEN (NL)
(74) Representative: Habets, Winand

(57) **Abstract**

The invention is in the field of predictive medicine. It provides means and methods for identifying compounds that cause or induce cholestasis. More in particular, the invention relates to an in vitro method of determining whether a compound has cholestatic properties by exposing an animal to a potentially cholestatic compound, extracting RNA from the animal, determining the level of expression of genes Pltp, Gsta2, Acot1, Cyp26a1, and Gck before and after exposure and concluding that the compound has a high probability of having cholestatic activity when the ratio of the level of expression of said genes after and before the exposure is above 2.0.

## Description

### Field of the invention

The invention is in the field of predictive medicine. It provides means and methods for identifying compounds that cause or induce cholestasis.

### Background of the invention

In medicine, cholestasis is a condition where bile cannot flow from the liver to the duodenum. The two basic distinctions are an obstructive type of cholestasis where there is a mechanical blockage in the duct system such as can occur from a gallstone or malignancy and metabolic types of cholestasis which are disturbances in bile formation that can occur because of genetic defects or acquired as a side effect of many medications.

From the viewpoint of cell biology, cholestasis is due to the disturbed transport of bile acids from the cytoplasm to the canaliculi. Bile formation is a secretory function of the liver. It begins in bile canaliculi that form between two adjacent surfaces of liver cells (hepatocytes) similar to the terminal branches of a tree. The canaliculi join each other to form larger and larger structures, sometimes referred to as Canals of Hering, which themselves join to form small bile ductules that have an epithelial surface. The ductules join to form bile ducts that eventually form either the right main hepatic duct that drains the right lobe of the liver, or the left main hepatic duct draining the left lobe of the liver. The two ducts join to form the common hepatic duct, which in turn joins the cystic duct from the gall bladder, to give the common bile duct. This duct then enters the duodenum at the ampulla of Vater.

Under a microscope, the individual hepatocytes will have a brownish-green stippled appearance within the cytoplasm, representing bile that cannot get out of the cell. Canalicular bile plugs between individual hepatocytes or within bile ducts may also be seen, representing bile that has been excreted from the hepatocytes but cannot go any further due to the obstruction. When these plugs occur within the bile duct, sufficient pressure (caused by bile accumulation) can cause them to rupture, spilling bile into the surrounding tissue, causing hepatic necrosis. These areas are known as bile lakes, and are typically seen only with extra-hepatic obstruction.

Cholestasis is commonly diagnosed when there is an elevation of both 5'-nucleotidase and ALP enzymes. With a few exceptions, the optimal test for cholestasis would be elevations of serum bile acid levels. However, this is not normally available in most clinical settings. The gamma-glutamyl transferase (GGT) enzyme was previously thought to be helpful in confirming a hepatic source of ALP; however, GGT elevations are markedly sensitive and lack the necessary specificity to be a useful confirmatory test for ALP. Normally GGT and ALP are anchored to membranes of hepatocytes and are released in small amounts in hepatocellular damage. In cholestasis, synthesis of these enzymes is induced and they are made soluble. GGT is elevated because it leaks out from the bile duct cells due to pressure from inside bile duct.

In a later stage of cholestasis AST, ALT and bilirubin may be elevated due to liver damage as a secondary effect of cholestasis.

New drug candidates are routinely tested for hepatotoxicity, usually in animals. This is ethically under pressure of the animal welfare groups but also is not reliable. One of the recent examples is the anti-diabetic compound troglitazone, which was withdrawn from the market.

According to the literature, the conventional method to study the effect of candidate compounds on bile secretion is to feed the compound to a group of animals, usually rats, and subsequently sacrifice the animals, excise the gall bladder filled with bile and determine its weight A decreased bile secretion (compound-induced cholestasis) predominantly represents a safety issue.

Conventional testing also is done by administering compounds to animals and subsequently measuring the bilirubin in the plasma. Bilirubin as a waist product of hemoglobin breakdown is modified by the liver and secreted via the bile and urine. Accumulation in the plasma indicates disturbed liver function and bile secretion.

It is also reported to use primary hepatocyte cultures taken from humans, rats and dogs. The cells are used, for example, in the analysis of differences between animal species in drug metabolism, the selection of suitable animal species for toxicology studies, the prior estimation of quantities and pathways involved in metabolism in humans or for determining the oral bioavailability of drug candidates. The final toxicology studies however, are performed in animals.

Yamanaka et al. (Proteomics. 2007 Mar;7(5):781-95) published the classification of genotoxic and non-genotoxic compounds by comparing the 2D-liver proteome profile after exposure in rats. This assay is thus performed in vivo and has limited throughput because of the proteomics application. It is not directed to classify compounds for cholestasis.

Mathijs et al. (Toxicol Sci. 2009 Dec;112(2):374-84) reported on the classification of genotoxic and non-genotoxic carcinogens by gene expression profiling in mouse primary hepatocytes. This assay is based on changes in mRNA concentrations instead of proteins. It is not specific for cholestasis.

The disadvantage of the conventional screening is the use of relatively large numbers of animals.

Apparently, there is a need for additional tests, that limit the use of laboratory animals, and that are more specific for a particular form of liver toxicity like cholestasis.

There remains a need in the art for reliable tests for cholestatic compounds.

### Summary of the invention

The invention relates to an in vitro method of determining whether a compound has cholestatic properties by exposing an animal to a potentially cholestatic compound, extracting RNA from the animal, determining the level of expression of genes Pltp, Gsta2, Acot1, Cyp26a1, and Gck before and after exposure and concluding that the compound has a high probability of having cholestatic properties when the ratio of the level of expression of said genes after and before the exposure is above 2.0.

### Detailed description of the invention

For development and evaluation of short term in vivo and/or in vitro models for hepatotoxicity, it is necessary to identify biomarkers reflective of later stage phenotypes such as cholestasis. Toxicogenomics can be used as a tool for biomarker identification allowing for faster screening on hepatotoxic properties of drugs or chemicals with reduced animal use. In this study, mechanism-based toxicogenomics is used as a tool to identify markers reflective of the onset and progression of cholestasis in C57BU6 mice using Cyclosporin A (CsA) as a model compound. Critical doses for toxicogenomic analysis were derived from a dose range finding study, in which increase in serum cholesterol, total bile acids and total bilirubin as well as induction of hepatocyte vacuolization 25 days upon repeated CsA administration through oral gavage were considered as critical effects. For toxicogenomic analysis to find early markers, livers of mice repeatedly treated with effect doses of CsA for one, four, and eleven days were collected. Gene expression changes were correlated to serum chemistry values of the individual mice to account for interanimal variation in severity of response. Gene set analysis was performed on the ranked list of correlated genes, resulting in the identification of gene sets related to the onset and progression of cholestasis. Two main gene set clusters include PPAR related processes and drug metabolism. Genes highly responsive to the severity of effect were identified and represent promising candidate biomarkers for onset and progression of CsA-induced cholestasis.

The objective of our study is to identify biomarkers reflective of the onset and progression of the hepatotoxic phenotype cholestasis in C57BL/6 mice using the model compound cyclosporin A (CsA). Mice were chosen as the model organism to contribute to the spectrum of species used in hepatotoxicity studies in vivo and in vitro. Important is the confirmation of the cholestasis phenotype based on serum chemistry and histopathology associated with cholestasis in a 25-day dose range finding study. It was hypothesized that correlation of gene expression to cholestasis-specific and coordinate increases in serum markers cholesterol (CHOL), total bilirubin (TBIL), and total bile acids (TBA) upon increasing dose and duration of treatment with CsA would result in identification of gene sets mechanistically related to the onset and progression of cholestasis.

In this study, mechanism-based toxicogenomic markers reflective of the onset and progression of drug-induced cholestasis were identified in CsA-treated mice. To achieve this, gene expression was correlated to increases in serum parameters of cholesterol, bilirubin, and bile acids, all hallmarks of cholestasis, resulting in ranking of gene lists according to severity of the phenotypic response in individual mice. Instead of comparing single genes in top correlated lists between cholesterol, bilirubin, and bile acids, individual gene information was compiled into gene sets. This allowed for enhanced functional comparison between serum parameters taking into account that different single genes between parameters might as well be part of similar gene sets (Kienhuis et al., 2009). For further analysis, gene sets were selected when significantly correlated to at least two serum parameters, as serum increases of only one bile constituent might not necessarily provide enough evidence to indicate cholestasis. Our findings show 55 gene sets, of which most are interconnected due to overlap of genes within gene sets. Clusters of interconnected gene sets represent gene set categories mechanistically associated with the cholestasis response. Deregulated genes within these correlated gene set categories will be discussed for their involvement in onset and progression of cholestasis and their possible application in alternative models to investigate the potential of drugs and chemicals to induce cholestasis. Serum chemistry and histopathology analyses performed by us showed establishment of cholestasis in mice 25 days upon treatment with CsA. Cholestasis is traditionally defined as the impairment of bile secretion, either due to a functional defect of hepatocytes or a defect at the bile duct level, resulting in retention of substances that are normally excreted into the bile (Zollner and Trauner, 2009; Ansede et al., 2010). CsA induces cholestasis by competitive inhibition of the bile salt export protein (Bsep) or Abcb11, which is the primary transporter involved in the biliary efflux of conjugated bile acids across the canalicular membrane (Bohan and Boyer, 2002; Ansede et al., 2010). In the clinic, cholestasis is diagnosed through measurement of serum values of the biliary constituents bile acids and bilirubin. In CsAinduced cholestasis, serum levels of these biliary constituents are increased (Ansede et al., 2010). This increase is confirmed in our study which showed gradual cholestasis development reflected by serum parameters that become more pronounced in time. Furthermore, at 25 days of exposure, formation of submembraneous vesicles is observed which suggests inhibited transport. Within our study design, we did not observe progression of cholestasis in terms of bile acid-induced hepatocyte toxicity resulting in leakage of AST and ALT enzymes into serum. This enables selection of time points and doses for identification of initial, sensitive and specific mechanisms involved in drug-induced cholestasis development, rather then processes beyond cholestasis induction. These mechanisms are of particular relevance for 13 short term in vivo and/or in vitro models were cholestasis is not expected to fully establish in a clinical sense. Many of the deregulated genes within the gene set categories PPAR related processes and drug metabolism that were correlated by our analysis to the cholestasis phenotype are involved in the currently known complex machinery of coordinated mechanisms activated upon cholestasis induction. Current knowledge on genes and proteins involved in the cholestasis mechanism is the result of extensive research in cell biology over the past years (Zollner and Trauner, 2009). Recent reviews describe that the disruption of bile homeostasis is immediately followed by an adaptive defense response aimed at reducing bile acid accumulation and concomitant toxicity (Zollner and Trauner, 2008; Zollner and Trauner, 2009; Wagner et al., 2010). Specific nuclear receptors (NRs) are at the basis of this adaptive response, which, after ligand activation, transcriptionally mediate target gene expression. The biliary constituents cholesterol, bilirubin and bile acids that accumulate in hepatocytes during onset and progression of cholestasis act as ligands for these NRs (Wagner et al., 2010). Hydrophobic bile acids are efficacious ligands for the Farnesoid X Receptor (FXR) (Parks et al., 1999) and the Pregnane X Receptor (PXR) (Staudinger et al., 2001). Bilirubin activates the Consitutive Androstane Receptor (CAR) and cholesterol and oxysteroid derivates are the main ligands for the Liver X Receptor a (LXRα) (Wagner et al., 2010). Involvement of the deregulated genes in our cholestasis model through their induction by biliary constituents via nuclear receptor activation will be described below. The classic bile acid biosynthetic pathway is initiated by CYP7A1, eventually metabolizing cholesterol to bile acids (Chiang, 2009). In our in vivo model, the negative correlation of Cyp7a1 mRNA expression to total bile acids is an important event in the adaptive response to the cholestasis phenotype, as it limits continuous bile acid production from cholesterol (Wagner et al., 2010). Cyp7a1 gene transcription is repressed by bile acid activated FXR through induction of the nuclear receptor small heterodimer partner (SHP) (Goodwin et al., 2000; Lu et al., 2000). Furthermore, FXR induces FGF15 in the ileum in mice (FGF19 in humans), which is an endocrine signal repressing expression of Cyp7a1 in the liver (Inagaki et al., 2005). Additionally, CYP7B1 constitutes and alternative pathway of conversion of cholesterol to oxysterols before further metabolism to bile acids (Chiang, 2009). Down-regulation of mRNA of this enzyme in our study is also in line with hepatoprotection to cholestasis. CAR is the proposed NR involved in Cyp7b1 gene regulation (Beilke et al., 2009), which might explain the correlation of Cyp7b1 expression to TBIL in our study. Another gene involved in cholestasis is Cyp17a1, which is a target gene of FXR and SHP and is involved in the steroidogenic pathway. Up-regulation renders increases in the enzymatic product, 17-hydroxyprogesterone (17-OHP), which showed to be able to induce a 14 cholestasis phenotype in wild type mice (Anakk et al., 2011). Down-regulation of Cyp17a1 as observed by us, suggests reduction of 17-OHP, preventing cholestasis as an adaptive response. Hepatic stellate cells (HSCs) are the major source of extracellular matrix deposition in the liver. Activated HSCs are associated with inflammation and fibrosis occurring upon liver injury. FXR activation by bile acids counteracts this process as it increases the expression of Pparg, returning the HSCs to their quiescent state (Fiorucci et al., 2004; Renga et al., 2011). Liver injury, however, is not phenotypically convincing based on results in our study, reflected by unchanged serum AST, ALT. However, histopathology in livers of some animals treated with the high dose of CsA for 1, 4 and 11 days was suggestive of subnecrotic hepatocellular fields. Still, increase in Pparg mRNA as shown by us might be explained by processes other than HSC activation. Overexpression of Pparg might also occur in hepatocytes. This is supported in our study by subsequent increase of two downstream targets, Me1 and Gck, both involved in adipocyte differentiation and lipid metabolism, suggesting tendency to adipogenic transformation of hepatocytes upon injury (Rogue et al., 2010). Deregulation of adipogenic pathways is supported by up-regulation of mRNA of other enzymes, namely Pltp and Fabp2. Pltp is a target gene of FXR (Urizar et al., 2000) and LXR (Cao et al., 2002), confirmed by its correlation to at least bile acids and cholesterol. Male Fabp2 knock-out mice were prone to develop fatty liver (Agellon et al., 2007), increase in Fabp2 expression in our study might therefore be an adaptive response to adipogenesis of hepatocytes. Generally, a direct role for PPARG, PLTP, and FABP2 in the regulation of cholestasis and bile acid homeostasis has not yet been reported. However, results in the present study might suggest gene expression counteracting a tendency towards adipogenesis of hepatocytes as a mechanism of cholestasis. Cholestatic liver disease is associated with disruption of vitamin A homeostasis in the liver, which is the main storage site of vitamin A in mammals (Hoeke et al., 2009; Wagner et al., 2010). The mRNA of two enzymes involved in vitamin A homeostasis, CYP26A1 and BCMO1, are both deregulated in our study. Up-regulation of Cyp26a1 increases retinoic acid degradation, whereas down-regulation of Bcmo1 prevents retinoic acid biosynthesis, eliminating vitamin A in the liver. A feedback process is involved, as the vitamin A derivative 9-cis retinoic acid acts as the ligand for the NR RARα, inducing expression of its target gene Cyp26a1. Again, HSCs, which are also known as vitamin A storing cells, might play a role in this process, as upon activation in cholestasis, these cells release their retinoic acid content (Hoeke et al., 2009). Regulation of mRNA of the typical bile acid and bilirubin detoxifying enzymes in mice, CYP3A11 and CYP2B10 (phase I) and UGT1A1 and SULT2A1 (phase II) (Wagner et al., 2005) are not observed in our study. However, mRNA of other phase II detoxifying 15 enzymes, Sult1a1 and numerous gluthathione-S-transferases (Gsts), are gradually upregulated in correlation with TBIL. This is not surprising, as TBIL activates CAR, an important regulator of all GSTs observed in our study (Knight et al., 2008). However, a possible role for these enzymes in detoxification of bile acids and bilirubin is not apparent from this study. GSTs have been proposed as proteins responsible for intracellular transport of bile acids. Although bile acid binding to GSTs was observed in vitro, results have not been confirmed in vivo (Agellon and Torchia, 2000). As can be concluded from the above, approximately one third of the deregulated genes identified by us can be related to the currently known mechanisms in induction of cholestasis and are all part of gene sets within the categories PPAR related processes and drug metabolism. The deregulated genes and corresponding gene sets not previously associated might provide new hypotheses on cholestasis development. Examples of such deregulated genes are the more than 2 times up-regulated Acot1 and the more than 2 times down-regulated Socs3 and Ddc. These proposed biomarkers are selected for their involvement in the significantly correlated gene sets tryptophan metabolism (Ddc), monocarboxylic acid metabolic process and cofactor metabolic process (Acot1), and the IL2RB pathway (Socs3). Ddc is involved in tryptophan metabolism, a gene set within the AA and CH metabolism gene set category. Further research is needed to confirm deregulation of these genes and gene sets in cholestasis and to identify their specific roles. From the pool of genes involved in mechanisms of cholestasis, that are mainly identified in basic cell biology research using knock out mouse models and findings in the clinic, the toxicogenomic strategy used here is successful in identifying genes that are deregulated during onset and progression of the phenotype. In particular these genes can serve as biomarkers for identification of cholestatic properties of drugs and chemicals. Therefore, genes involved in PPAR related processes and drug metabolism represent promising candidate biomarkers. It is also shown that the important initiators of the cholestatic response, the nuclear receptors and the specific bile acid transporters like Bsep, do not necessarily represent appropriate biomarkers, as they are not gradually modulated in the early cholestasis response induced by CsA. To investigate the relevance of the responsive biomarkers for cholestasis induction in humans, a concordance model for interspecies and in vitro to in vivo comparison can be used (Kienhuis et al., 2011). In particular the study performed by Ellinger- Ziegelbauer et al. (2011) using toxicogenomics to identify cholestasis biomarkers in rats can be used for mouse-rat interspecies comparison (Ellinger-Ziegelbauer et al., 2011). In their study, a similar approach combining transcriptomic data with phenotypic measures, namely, bile duct epithelial cell necrosis, hyperplasia and/or increased bilirubin and cholestasis, was used. In contrast to our study, however, the severity of histopathological responses observed in rats upon compound treatment was much higher. Therefore, gene sets obtained by Ellinger16 Ziegelbauer et al. (2011) might be more reflective of successive stages beyond onset and progression, evidenced by the acute phase response, initiation and execution of cell death, early inflammatory response and tissue regeneration. Similar to our results, gene regulation suggesting HSC activation initiating fibrosis was observed. Furthermore, the adaptive response to cholestasis reflected by deregulation of genes with well-known functions in bile homeostasis was apparent. Results in our study might therefore add to this, showing gene set regulation reflective of onset and progression. These processes proceed to establishment of cholestasis and inflammation, regeneration and cell death as processes beyond cholestasis, observed in the rat toxicogenomics study (Ellinger-Ziegelbauer et al., 2011). Although gene sets identified in mice seem relevant for primary mechanisms of cholestasis induction in mammals, or possibly vertebrates, in general, differences in bile acid homeostasis and cholestasis development between mice and humans is important to consider the applicability of mice as a model species. In contrast to the hydrophobic bile acid pool in humans, under physiological conditions the bile acid pool in mice is mostly composed of hydrophilic bile acids, suggesting a less important role of FXR and PXR in normal bile homeostasis in mice compared to humans (Chiang, 2009). Apart from species differences in bile acid composition and transporter gene regulation, a major obstacle for a direct extrapolation is the different duration of cholestasis in animal models (days to weeks) versus human cholestatic disorders (weeks to years or even decades) (Trauner et al., 2005). Therefore, results from studying bile acid synthesis in the mouse (and rat) models may not be extrapolated to humans without verification in suitable human models (Chiang, 2009). However, the objective of our study is to identify mechanistic markers for onset and progression of cholestasis and not for bile acid homeostasis and endpoints of disease or possible targets for treatment. Gene expression changes reflecting early, adaptive responses might therefore still be representative for cholestasis induction in humans and might allow for screening of drugs and chemicals for cholestatic properties in animal or alternative models. In conclusion, this study is the first to identify biomarkers that show a gradual response in gene expression along with increasing cholestasis-related serum parameters reflective of onset and progression of CsA-induced cholestasis in C57BL/6 mice. In particular the responsiveness of gene sets and genes is of relevance to label them as biomarkers, as this feature allows for identification of cholestasis-inducing properties of drugs and chemicals early in short-term rodent in vivo models or in vitro models. Mechanistically confirmed gene sets include PPAR related processes and drug metabolism, with specific deregulated genes confirmed to be involved in the adaptive response to cholestasis in the liver. Correlation to increases in biliary constituents in serum allows for generalization of these findings to druginduced cholestasis rather than restriction to CsA. Further application of the concordance model to integrate cholestasis-induced expression profiles between vertebrates in vivo, which 17 may include rodents as well as zebrafish(embryo's), and hepatocyte-based models in vitro of rodent and/or human origin will reveal whether biomarkers found in this study remain valid for drug-induced cholestasis through Bsep inhibition as well as other types.

Hence the invention relates to a method of determining whether a compound has cholestatic properties by exposing an animal to a potentially cholestatic compound, extracting RNA from the animal, determining the level of expression of genes Pltp, Gsta2, Acot1, Cyp26a1, and Gck before and after exposure and concluding that the compound has a high probability of having cholestatic properties when the ratio of the level of expression of said genes after and before the exposure is above 2.0.

The reliability of the assay could be improved when the genes Socs3, Ddc and Cyp7b1 were included in the method as described above. These genes are predictive for the cholestatic properties of a compound when the ratio before and after is below -2.0.

### Examples

### Example 1: MATERIALS AND METHODS

Compound. Cyclosporin A, CAS-no 59865-13-3; purity minimum 98%, was kindly provided by Novartis, Basel, Switzerland.

Animals. Male C57BL/6JIco mice, aged 10 weeks at the start of the treatment period (21-27 g), were obtained from Charles River GmbH, Sulzfeld, Germany. Animals were kept under controlled specific pathogen-free conditions (23oC, 40%-50% humidity) under a 12-hour light-dark cycle (7:00 am-7:00 pm), and housed in groups of five. Food and tap water were available ad libitum during the whole experiment. Experiments were conducted at the animal facility of the Leiden University Medical Center, and carried out in accordance with Dutch law.

Animal treatment. For the dose range finding study, forty animals were assigned to eight groups of five mice per group. For the array study, sixty animals were assigned to twelve groups of five mice per group. Mice were dosed by oral gavage (p.o.) in a volume of 4 ml/kg BW CsA in olive oil for five times a week between 2:00 and 4:00 pm. Treatment doses in the array study were based on the dose range finding study in which CsA doses were determined that induced cholestasis parameters after 25 days of treatment. Doses are presented in Table 1. Animals were sacrificed by inhalation of CO2 and heart punction at one, four, and eleven days post CsA administration. Time of autopsy was done between 2:00 and 4:00 pm. Blood was collected in 0.8 mL Minicollect serum collection tubes (Greiner Bio-One, Alphen aan de Rijn, The Netherlands) for serum chemistry analyses. The liver was immediately dissected. For the array study, the central half of the left lateral lobe was collected in RNAlater (Applied Biosystems / Ambion, Nieuwerkerk a/d IJssel, The Netherlands) and kept for a maximum of one week at 4oC and frozen at -80oC until use for RNA isolation. For both the dose range finding study and the array study, the remaining part and a part of the right liver lobe were kept aside in 4% neutral buffered formalin for pathological examination.

Serum chemistry. Serum alanine aminotransferase (ALT) activity, aspartate aminotransferase (AST) activity, cholesterol (CHOL), total bilirubin (TBIL) and total bile acids (TBA) were analyzed on a Beckman Coulter LX20 Clinical Chemistry Analyzer using Beckman reagent kits (Beckman Coulter B.V., Woerden, The Netherlands) for ALT, AST, TBIL, and CHOL, and a Dialab reagent kit (DIALAB GmbH, Neudorf, Austria) for TBA.

Histopathology. After 24 hours fixation in 4% neutral buffered formalin, liver samples were maintained in 70% ethanol until further processing, which included automated dehydration, then embedding in paraffin, sectioning at 5 microns, and staining with hematoxylin and eosin.

Statistical analysis. Critical effect doses of serum chemistry parameters and histopathology in the dose range finding study were determined using bench mark dose (BMD) approach with the PROAST software ((Slob, 2002); www.rivm.nl/proast). Differences between serum 6 levels in treated versus non-treated animals in the array study were statistically significant at a P value below 0.01, determined by ANOVA followed by Dunnett's test.

GeneChip hybridization. For RNA analysis, RNA later stabilized liver samples were homogenized with the buffer RNeasy Lysis Buffer (RLT) supplied in the RNeasy Mini Kit (Qiagen, Leusden, The Netherlands). Total RNA isolation was performed using the RNeasy Mini Kit with the QIACUBE (Qiagen, Leusden, The Netherlands). RNA concentrations, quality and integrity were determined using the Nanodrop and the BioAnalyzer (Agilent Technologies, Palo Alto, CA). All samples contained intact total RNA with RIN values > 8. Sample labeling and Affymetrix hybridizations were performed by ServiceXS B.V. (Leiden, the Netherlands). The Affymetrix 3'IVT-Express Labeling Kit (#901229, Affymetrix, Santa Clara, California, USA) was used to synthesize Biotin-labeled cRNA. From each sample 100 ng was used as input for the labeling reactions. Fragmentation of the cRNA was performed using 7.5 µg. Subsequently, the hybridization cocktail was prepared according to the manual of the Affymetrix 3'IVT Express Labeling Kit with 4.5 µg of fragmented cRNA and hybridized to the Affymetrix HT Mouse Genome 430 PM Array. The Hybridization, Wash and Stain Kit (#901530) was used for the hybridizations, washing, staining and scanning of the chips according to Affymetrix protocols. Samples were randomly hybridized on the arrays.

Data preprocessing and quality control. Cell Intensity Files (*.cel) generated for each GeneChip using the Affymetrix GeneChip Command Console (v3.0) software were normalized using the Robust Multichip Average (RMA) algorithm (Irizarry et al., 2003) and the MBNI custom CDF for this chip (http://brainarray.mbni.med.umich.edu/Brainarray/Database/CustomCDF/CDF/). Output consisted of 16492 probe sets corresponding to unique Gene IDs. Quality control consisted of the various RMA QC parameters as well as visual inspection of residual plots created in RMAexpress (Bolstad et al., 2003). Two out of 64 samples did not meet the quality criteria and were therefore removed from further analysis.

Functional correlation of gene expression and serum parameters. Raw gene expression data were transformed to the natural logarithm (Ln) using R (version 2.10.0). Ln-transformed data were correlated to serum values of CHOL, TBA and TBIL by performing Spearman correlation analysis using Babelomics (Medina et al., 2010). Genes were ranked based on the correlation coefficient. The top contained the highest positively correlated genes and the bottom contained the highest negatively correlated genes. The ranked gene list was then analyzed for enriched gene sets using the GSEApreranked option in the Gene Set Enrichment Analysis (GSEA) software, v.2.0 (Subramanian, 2002). 7

Molecular concepts analysis. From the GSEA analysis, significantly correlated gene sets overlapping between at least two parameters were selected. From these gene sets, for CHOL, TBIL, and TBA each, the top 20% ranked genes were selected. Subsequently, information on size of gene sets and significant overlap between top 20% genes within gene sets was generated using Venn Mapper V1.01 (Smid et al., 2003). For molecular concepts analysis (Rhodes et al., 2007), a network description file was generated showing gene sets significantly correlated with CHOL, TBA and/or TBIL (as nodes) and significantly overlapping top 20% genes (P<0.05) (as edges). The resulting network was visualized in cytoscape, version 2.7.0 (Cline et al., 2007).

Deregulated genes involved in gene set categories. From the subset of genes underlying significantly correlated gene sets, deregulated genes were selected based on a minimum fold change difference of 1.5 between high responsive and low responsive mice for each serum parameter. Selection of responsive mice was based on a 95% percentile cut-off per parameter. Low responders represent animals with serum values below the 5% percentile, high responders represent animals with serum values above the 95% percentile. Fold change differences between low and high responders were calculated based on the median expression values within the low and high responder groups.

### Example 2:Dose range finding study

Serum chemistry and histopathology. In the dose range finding study critical effect doses (CEDs) of 1.32 mg/kg BW for cholesterol, 23.7 mg/kg BW for total bilirubin, and 0.564 mg/kg BW for total bile acids were derived. No effect doses were obtained for ALT and AST. BMD analysis of histopathology results retrieved the dose where the average animal was diagnosed with strong vacuolization, which was at 0.631 mg/kg BW. For the array study, three doses were selected that exceeded the CED of at least three out of four parameters (Table 1). Doses of 3.0 and 8.9 mg/kg BW exceeded the CED for cholesterol and total bile acids, as well as strong vacuolization. The dose of 26.6 mg/kg BW exceeded the CED of all parameters.

**Table 1 Effect doses for serum chemistry and histopathology in dose range finding study. -,doses below critical effect dose; +, doses above critical effect dose.**

| DRF doses (mg/kg bw) | Serum chemistry | | | | | Histopathology |
|---|---|---|---|---|---|---|
| | CHOL | TBIL | TBA | AST | ALT | Strong vacuolization |
| **0.0** | - | - | - | - | - | - |
| 0.1 | - | - | - | - | - | - |
| 0.3 | - | - | - | - | - | - |
| 1.0 | - | - | + | - | - | + |
| **3.0** | + | - | + | - | - | + |
| **8.9** | + | - | + | - | - | + |
| **26.7** | + | + | + | - | - | + |
| 80.0 | + | + | + | - | - | + |

### Example 3: Toxicogenomics study

Serum chemistry and histopathology. In any dose*time group, CsA exposure did not result in increases in AST and ALT (data not shown). The plotted serum values per animal for CHOL, TBIL, and TBA were plotted.. Dose and time responsive trends in increases were observed for each parameter. Increases were significant (p<0.01, ANOVA followed by Dunnett's test) for CHOL upon treatment with the high dose for 1 and 11 days and for TBIL upon treatment with the medium and high dose for 11 days. One of the vehicle treated mice in the 11 day treatment group, mouse 43, was considered an outlier and was excluded from further analysis. No submembraneous vacuolization was observed in any of the dose groups 1, 4 and 11 days after CsA exposure. Histopathology in livers of 4, 3, and 1 animal(s) was suggestive of 9 subnecrotic hepatocellular fields upon treatment with the high dose of CsA for 1, 4, and 11 days, respectively. No further remarkable observations were made.

Correlation analysis. The top 5 and bottom 5 ranking of CHOL, TBIL, and TBA measurements in individual mice according to correlation to gene expression were plotted. In general, controls and 1 day treatments were overrepresented in the upper half of the figures for CHOL, TBIL, and TBA where serum values are low, whereas high dose and 11 day exposures were overrepresented in the lower half of the figures where serum values are high. This shows that correlation of gene expression values with serum values of individual mice does not necessarily result in clusters of similar dose*time combinations.

Gene set analysis. GSEA on the ranked lists of genes from the correlation analysis resulted in identification of 66, 68, and 66 pathways significantly correlated to CHOL, TBIL, and TBA, respectively. For further analysis, pathways significantly correlated to at least two cholestasis-related serum parameters were considered relevant.

Deregulated genes involved in gene set categories. The subset of deregulated genes with a fold change of more than 2.0 up- or down-regulation in the high responder versus the low responder group obtained from these genes are shown in Table 2.

**Table 2: Genes are ranked according to fold change of high responders versus low responders based on the highest correlated serum parameter. Positive fold changes represent up-regulation; negative fold changes represent down-regulation. chol, cholesterol; tbil, total bilirubin; tba, total bile acids; HR, high responders; LR, low responders.**

| **Entrez ID** | **Gene Symbol** | **Highest Correlation** | **Fold change HR vs LR** |
|---|---|---|---|
| 18830 | *Pltp* | chol | 3,7 |
| 14858 | *Gsta2* | tbil | 3,2 |
| 26897 | *Acot1* | tba | 2,7 |
| 13082 | *Cyp26a1* | tbil | 2,4 |
| 103988 | *Gck* | chol | 2,3 |
| 12702 | *Socs3* | chol | -2.1 |
| 13195 | *Ddc* | tbil | -2.3 |
| 13123 | *Cyp7b1* | tbil | -2.4 |

## Claims

1. In vitro method of determining whether a compound has cholestatic properties by exposing an animal to a potentially cholestatic compound, extracting RNA from the animal, determining the level of expression of genes Pltp, Gsta2, Acot1, Cyp26a1, and Gck before and after exposure and concluding that the compound has a high probability of having cholestatic properties when the ratio of the level of expression of said genes after and before the exposure is above 2.0.

2. Method according to claim 1 wherein additionally the level of expression of genes Socs3, Ddc and Cyp7b1 is determined and wherein the ratio of the level of expression of said genes after and before the exposure is below -2.0.
